# EUROPÄISCHE PATENTSCHRIFT

(11) **EP 1 967 145 B1**
(45) Veröffentlichungstag und Bekanntmachung des Hinweises auf die Patenterteilung: **19.10.2011**
(21) Anmeldenummer: 08151399.6
(22) Anmeldetag: 14.02.2008
(51) Int. Cl.: A61B 17/16

(54) **Chirurgische Knochenstanze**
Surgical bone punch
Poinçonneuse d'os chirurgicale

(30) Priorität: 09.03.2007 DE 102007011670
(43) Veröffentlichungstag der Anmeldung: 10.09.2008
(73) Patentinhaber: Aesculap AG, 78532 Tuttlingen (DE)
(72) Erfinder: Faulhaber, Konstantin, 78665, Frittlingen (DE); Schulz, Peter, 79843, Löffingen (DE); Nesper, Markus, 78532, Tuttlingen (DE); Weisshaupt, Dieter, 78194, Immendingen (DE)
(74) Vertreter: Hoeger, Stellrecht & Partner Patentanwälte

(56) Entgegenhaltungen:
- WO-A-99/08604
- DE-U1-202004 015 643
- US-A- 3 752 161
- US-A- 5 653 713
- US-A1- 2004 186 499

## Beschreibung

Die Erfindung betrifft eine chirurgische Knochenstanze mit einem mit einem Handstück verbundenen stationären Schaft, einem an diesem stationären Schaft längsverschieblich gelagerten Schiebeschaft und mit einem motorischen Antrieb im Handgriff zum Verschieben des Schiebeschaftes aus einer proximalen Ruhestellung in eine distale Arbeitsstellung mit einer vorgegebenen Vorschubkraft.

Eine solche chirurgische Knochenstanze mit einem pneumatischem Antrieb ist beispielsweise aus der DE 20 2004 015 643 U1 bekannt. Der pneumatische Antrieb im Handgriff erleichtert dem Operateur die Arbeit und führt zu reproduzierbaren Vorschubkräften, die für den Handgriff charakteristisch sind, die aber andererseits sich nicht an unterschiedlich dimensionierte Einheiten aus stationärem Schaft und Schiebeschaft anpassen lassen. Wenn derartige Einheiten aus stationärem Schaft und Schiebeschaft eingesetzt werden, die unterschiedlich dimensioniert sind, beispielsweise die unterschiedlich breite Knochenschneiden aufweisen, dann können die pro Längeneinheit der Knochenschneide auftretenden Schneidkräfte daher bei schmalen Schneiden so groß werden, dass Beschädigungen der Schneiden oder Verletzungen des Gewebes auftreten, die unerwünscht sind.

Es ist daher bei bekannten Knochenstanzen vorgesehen, dass die Vorschubkräfte des Antriebes eingestellt werden können, beispielsweise durch die Umschaltung auf unterschiedliche Einlassventile für das pneumatische Betätigungsmedium im Handgriff. Dies ist aber insbesondere beim Auswechseln der Einheiten aus stationärem Schaft und Schiebeschaft umständlich für den Operateur, und es besteht die Gefahr, dass diese Umschaltung vergessen wird und dass dann die Knochenstanze mit einer Vorschubkraft betrieben wird, die der jeweils verwendeten Einheit aus stationärem Schaft und Schiebeschaft nicht angemessen ist.

US 3 752 161 zeigt eine Knochenstanze mit den Merkmalen des Oberbegriffs von Anspruch 1.

Es ist Aufgabe der Erfindung, eine chirurgische Knochenstanze der eingangs beschriebenen Art derart auszugestalten, dass sich die Vorschubkraft einer Einheit aus stationärem Schaft und Schiebeschaft automatisch an die notwendige Größe anpasst, ohne dass dazu eine Veränderung der von dem motorischen Antrieb gelieferten Vorschubkraft notwendig wird.

Diese Aufgabe wird bei einer chirurgischen Knochenstanze der eingangs beschriebenen Art erfindungsgemäß dadurch gelöst, dass der Schiebeschaft einen Anschlag trägt, der beim Verschieben des Schiebeschaftes in die Arbeitsstellung an einem elastischen Federelement anschlägt, das sich am stationären Schaft abstützt und beim Verschieben des Schiebeschaftes der Vorschubkraft des Antriebes entgegenwirkt.

Durch diese der Vorschubkraft des Antriebes entgegen wirkende Kraft des elastischen Federelementes wird die resultierende Vorschubkraft herabgesetzt, die auf die Schneide der Knochenstanze wirkt, und durch entsprechend dimensionierte elastische Federelemente kann ausgewählt werden, wie stark diese Reduktion ist, d.h. die Reduktion kann so gewählt werden, dass bei jeder Schneide die gewünschte Vorschubkraft auftritt, wobei der motorische Antrieb weiterhin immer die volle und gleichbleibende Vorschubkraft liefert, die durch das elastische Federelement jedoch mehr oder weniger reduziert wird.

Erfindungsgemäß ist vorgesehen, dass der Anschlag des Schiebeschaftes, das elastische Federelement und die Abstützung des elastischen Federelementes am stationären Schaft so angeordnet und dimensioniert sind, dass das elastische Federelement bei der Verschiebung des Schiebeschaftes aus der Ruhestellung in die Arbeitsstellung erst nach einem Teil des Verschiebeweges wirksam wird und der Vorschubkraft des motorischen Antriebs entgegenwirkt. Damit erhält man bei dem ersten Teil der Vorschubbewegung in jedem Fall die volle Vorschubkraft des motorischen Antriebes, eine Reduktion setzt erst nach einem Teil des Verschiebeweges ein und wird erst dann wirksam, wenn die Schneiden der Knochenstanze sich aneinander annähern und dabei durch zu hohe Vorschubkräfte beschädigt werden könnten.

Bei einer bevorzugten Ausführungsform kann vorgesehen sein, dass der stationäre Schaft eine Aufnahmekammer für ein als Druckfeder ausgebildetes elastisches Federelement aufweist, in die ein am Schiebeschaft angeordneter Anschlag eintaucht, der beim Vorschieben des Schiebeschaftes die Druckfeder komprimiert.

Besonders vorteilhaft ist es, wenn der stationäre Schaft mit dem Schiebeschaft lösbar mit dem Handgriff verbunden und von diesem abnehmbar ist. Es ist auf diese Weise möglich, auf demselben Handgriff unterschiedliche Einheiten aus stationärem Schaft und Schiebeschaft aufzusetzen und damit die Knochenstanze an die jeweiligen Gegebenheiten anzupassen.

Bei einer besonders bevorzugten Ausführungsform ist vorgesehen, dass der Knochenstanze ein Satz von mehreren auswechselbaren stationären Schäften mit Schiebeschäften zugeordnet ist, von denen mindestens ein Teil verschieden dimensionierte und mit unterschiedlichen Vorschubkräften einsetzbare Schneiden trägt, und dass jede Einheit aus stationärem Schaft und Schiebeschaft entweder kein elastisches, der Vorschubkraft entgegenwirkendes elastisches Federelement oder ein elastisches Federelement mit unterschiedlicher Federcharakteristik aufweist, so dass die vom Antrieb erzeugte Vorschubkraft vollständig bzw. entsprechend der jeweiligen Federcharakteristik unterschiedlich reduziert auf die Schneide oder die Schneiden einwirkt.

Bei einer solchen Ausgestaltung kann der Operateur je nach Bedarf unterschiedliche Einheiten aus stationärem Schaft und Schiebeschaft auf den Handgriff aufsetzen und es ergeben sich zwangsläufig Begrenzungen der Vorschubkräfte, die an die Dimensionierung der jeweiligen Einheiten individuell angepasst sind, d.h. jede Einheit wird mit der für diese Einheit optimalen maximalen Vorschubkraft in die Arbeitsstellung verschoben. Dazu ist keinerlei Auswahltätigkeit oder Umstellung seitens des Operateurs notwendig, der Handgriff kann immer mit derselben, vorgegebenen Vorschubkraft arbeiten.

Insbesondere kann vorgesehen sein, dass die Schneide oder die Schneiden verschiedener Einheiten unterschiedlich breit sind.

Knochenstanzen können an jedem der beiden Teile, also am stationären Schaft und am Schiebeschaft jeweils eine Schneide tragen, es ist aber auch möglich, dass nur eines dieser Teile eine Schneide trägt und das andere eine als Amboss wirkende Anlagefläche für Gewebe, gegen die die Schneide vorgeschoben wird.

Die nachfolgende Beschreibung bevorzugter Ausführungsformen der Erfindung dient im Zusammenhang mit der Zeichnung der näheren Erläuterung. Es zeigen:
- Figur 1:: eine Seitenansicht einer Knochenstanze mit einem Handgriff und mit einer darauf aufgesetzten Einheit aus stationärem Schaft und Schiebeschaft;
- Figur 2:: eine Ansicht ähnlich Figur 1 mit der Einheit aus stationärem Schaft und Schiebeschaft in einer gegenüber dem Handgriff ausgeschwenkten Lösestellung;

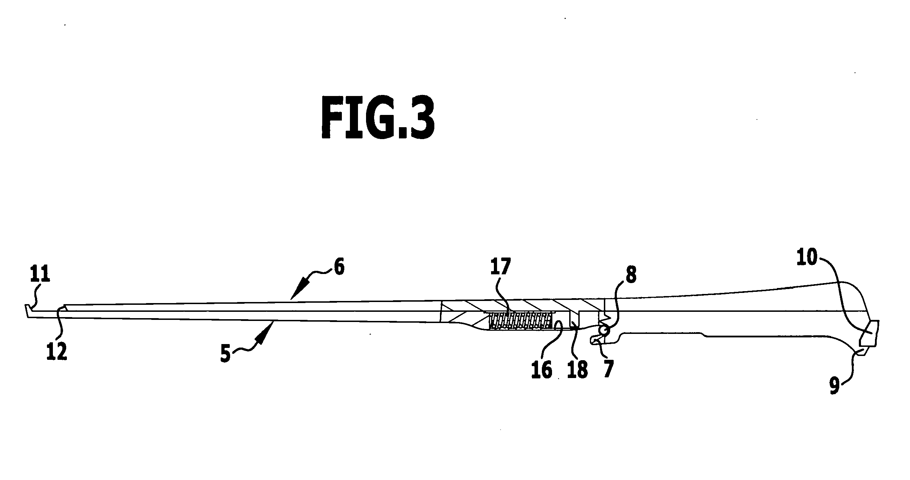

- Figur 3:: eine Teilschnittansicht einer Einheit aus stationärem Schaft und Schiebeschaft mit einer Aufnahmekammer für eine Druckfeder im stationären Schaft;

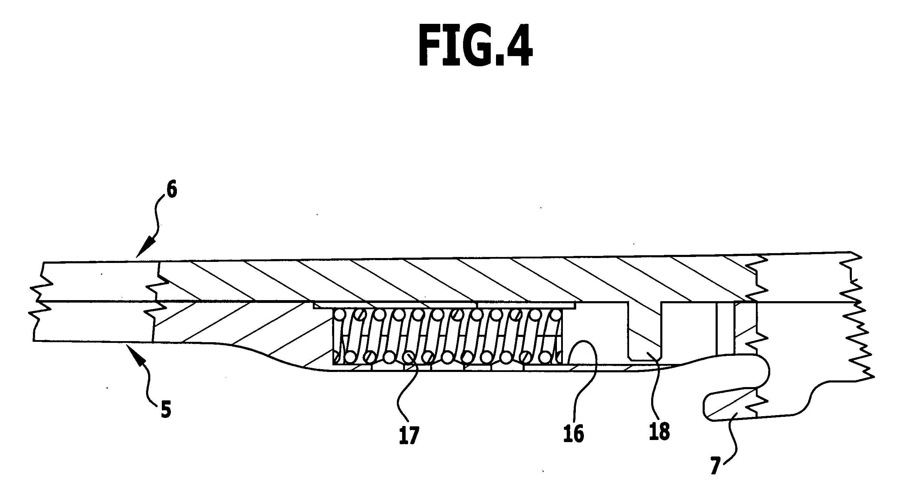

- Figur 4:: eine vergrößerte Detailansicht der Aufnahmekammer und der Druckfeder und
- Figur 5:: ein Diagramm zur Beschreibung der Größe der effektiven Vorschubkraft des Schiebeschaftes in Abhängigkeit vom Verschiebeweg.

Die in der Zeichnung dargestellte Knochenstanze 1 umfasst einen Handgriff 2 mit einem Anschluss 3 an seinem unteren Ende, an den eine in der Zeichnung nicht dargestellte Druckluftleitung angeschlossen werden kann. Im Inneren des Handgriffs 2 befindet sich ein durch Druckluft betriebener motorischer Antrieb, der ebenfalls nicht dargestellt ist und der mittels eines in den Handgriff einschiebbaren Betätigungsgriffes 4 aktiviert werden kann.

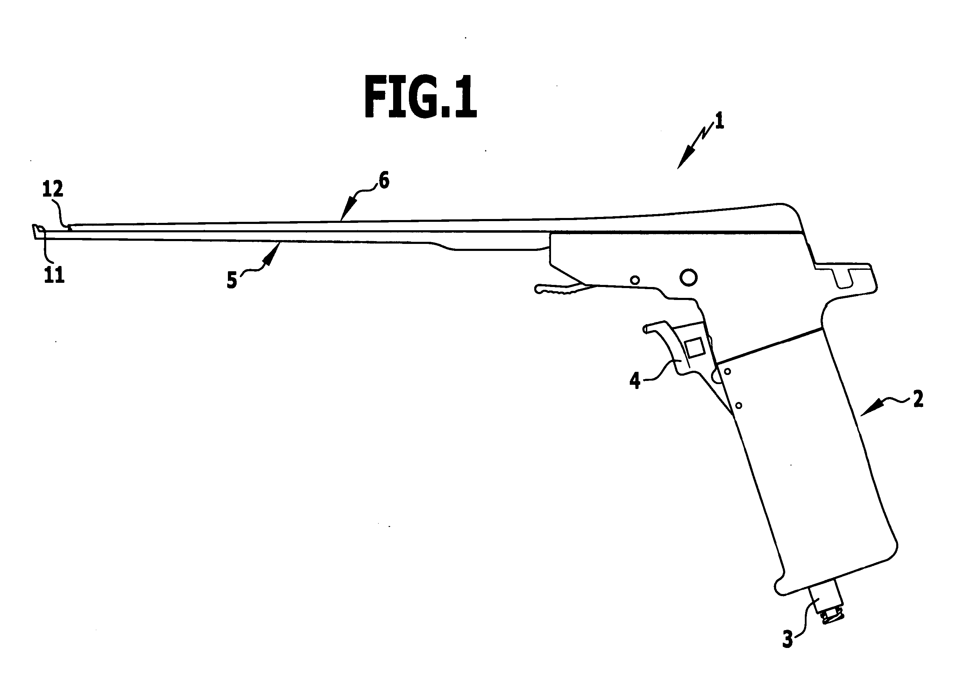

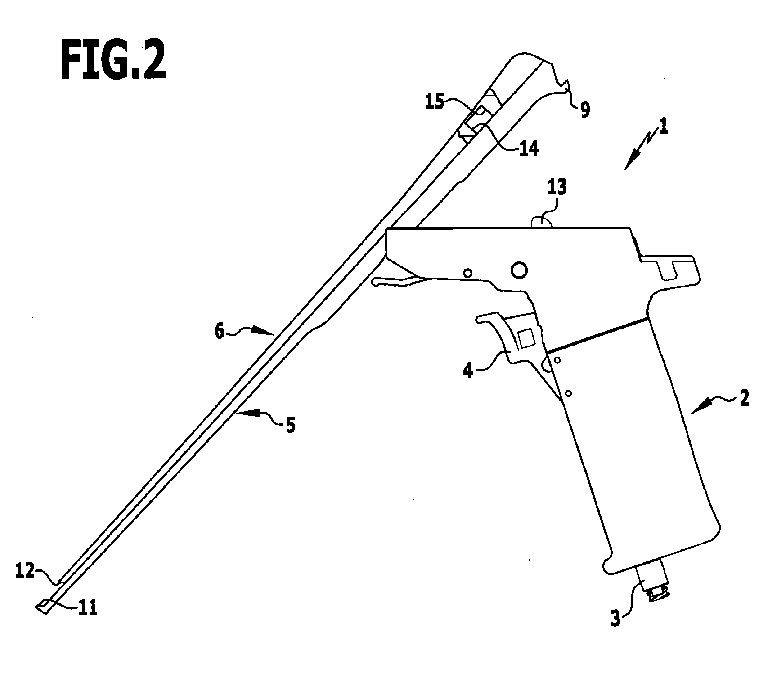

An dem Handgriff 2 ist lösbar eine Einheit aus einem stationären Schaft 5 und einem daran längsverschieblich gelagerten Schiebeschaft 6 gehalten. Zur Festlegung dieser Einheit am Handgriff 2 trägt der stationäre Schaft 5 an seinem unteren Ende einen Haltehaken 7, der zum distalen, vorderen Ende des stationären Schaftes 5 hin offen ist und einen Haltesteg 8 am oberen distalen Ende des Handgriffes 2 umgreift. Dieser Haltesteg 8 bildet eine Schwenkachse für den stationären Schaft 5 aus. Um diese Schwenkachse kann der stationäre Schaft 5 zwischen einer Lösestellung, in der er gegenüber der Oberseite des Handgriffes 2 schräg verläuft (Figur 2), und einer Fixierstellung verschwenkt werden, in der der stationäre Schaft 5 parallel zur Oberseite des Handgriffes 2 verläuft (Figur 1). In der Fixierstellung untergreift der stationäre Schaft 5 mit einer an seinem proximalen unteren Ende angeordneten Haltenase 9 einen Rastvorsprung 10 am oberen, proximalen Ende des Handgriffes 2, so dass der stationäre Schaft 5 dadurch am Handgriff 2 festgelegt wird. Der Rastvorsprung 10 kann durch geeignete, in der Zeichnung nicht näher dargestellte Mittel zurückgezogen werden, so dass die Haltenase 9 freigegeben wird. Auf diese Weise kann die Einheit aus stationärem Schaft 5 und Schiebeschaft 6 in einfacher Weise von dem Handgriff 2 abgenommen und durch eine andere Einheit ersetzt werden.

Der stationäre Schaft 5 ist ein sich vom Handgriff 2 zu seinem distalen Ende erstreckender, starrer Schaft, der an seinem distalen Ende eine schräg nach oben weisende Anschlagfläche 11 trägt. An dem stationären Schaft 5 ist in einer Führung längsverschieblich geführt der Schiebeschaft 6 gelagert, der sich ebenfalls im wesentlichen über die gesamte Länge des stationären Schaftes 5 erstreckt und an seinem distalen Ende in einer Schneide 12 endet, die der Anschlagfläche 11 gegenübersteht. Beim Annähern der Schneide 12 an die Anschlagfläche 11 kann zwischen der Schneide 12 und der Anschlagfläche 11 angeordnetes Gewebe, beispielsweise Knochenmaterial, durchschnitten werden.

Die Verschiebung des Schiebeschaftes 6 längs des stationären Schaftes 5 erfolgt über den motorischen Antrieb im Inneren des Handgriffes 2. Dieser motorische Antrieb verschiebt einen Mitnehmer 13 in Richtung des Schiebeschaftes und zurück, er ragt nach oben geringfügig über die Oberseite des Handgriffes 2 hervor und umgreift von unten her durch den stationären Schaft hindurch den Schiebeschaft 6. Dieser Schiebeschaft 6 trägt unmittelbar vor und unmittelbar hinter dem Mitnehmer 13 seitliche Anschlagflächen 14, 15, die an dem Mitnehmer 13 anliegen und dadurch bei dessen Vor- und Rückbewegung den Schiebeschaft 6 mitnehmen. Beim Druck auf den Betätigungsgriff 4 und der Aktivierung des motorischen Antriebes wird der Schiebeschaft 6 in distaler Richtung verschoben, beim Beenden der Aktivierung wird der Mitnehmer 13 in die Ausgangslage zurück bewegt, entweder durch den motorischen Antrieb oder durch geeignete Federmittel im Handgriff, so dass der Schiebeschaft wieder in die proximale Ausgangsstellung zurückgezogen wird.

In dem stationären Schaft 5 ist vor dem Haltehaken 7 eine nach oben hin offene Aufnahmekammer 16 angeordnet, in die eine Schraubenfeder 17 eingelegt ist. Diese stützt sich am distalen Ende der Aufnahmekammer 16 an dem stationären Schaft 5 ab und sie ist kürzer als die Aufnahmekammer 16, so dass sie lediglich den distalen Teil der Aufnahmekammer 16 ausfüllt, beispielsweise kann die Schraubenfeder 17 halb so lang sein wie die Aufnahmekammer.

In den von der Schraubenfeder 17 nicht ausgefüllten Teil der Aufnahmekammer 16 taucht ein am unteren Ende des Schiebeschaftes 6 angeordneter Vorsprung 18 ein, der beim Vorschieben des Schiebeschaftes 6 aus der Ruhestellung in die distale Arbeitsstellung zunächst den von der Schraubenfeder 17 freien Teil der Aufnahmekammer durchläuft und dann an der Schraubenfeder 17 zur Anlage kommt. Bei der weiteren Verschiebung in distaler Richtung wird die Schraubenfeder 17 durch diesen Vorsprung 18 komprimiert und übt dadurch bei fortschreitender Verschiebung in distaler Richtung eine zunehmende Rückstellkraft auf den Schiebeschaft aus.

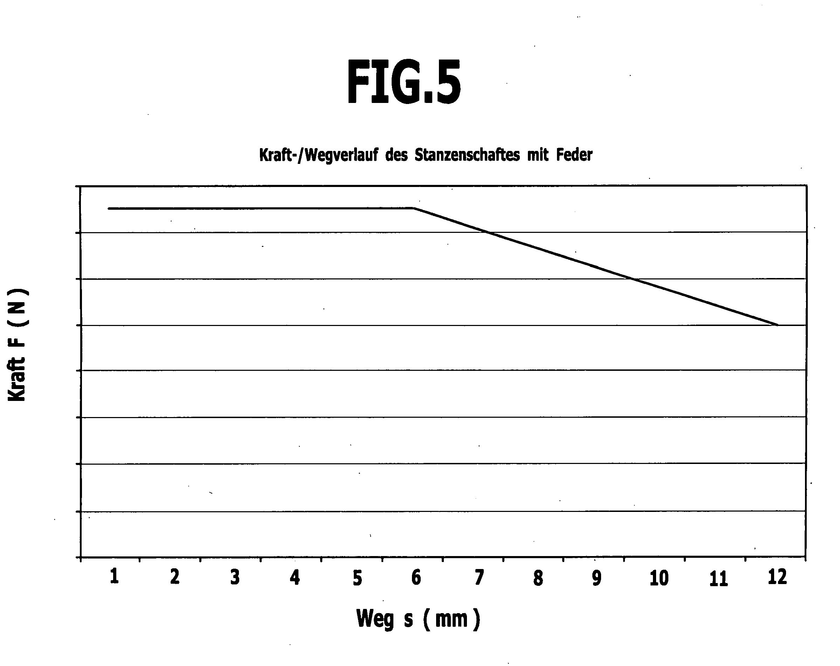

Die Vorschubkraft des motorischen Antriebes bleibt während der gesamten Vorschubbewegung im wesentlichen konstant, so dass die effektiv den Schiebeschaft 6 in distaler Richtung verschiebende Vorschubkraft beim Beginn der Vorschubbewegung der Vorschubkraft des motorischen Antriebes entspricht und dann nach dem Anlegen des Mitnehmers 13 an der Schraubenfeder 17 kontinuierlich abnimmt. Dieser Kraftverlauf ist in Figur 5 dargestellt.

Die Charakteristik der in die Aufnahmekammer 16 eingelegten Schraubenfeder 17 wird angepasst an die Dimensionierung der jeweiligen Einheit aus stationärem Schaft 5 und Schiebeschaft 6, beispielsweise wird bei einer sehr schmalen Schneide, beispielsweise einer Schneide mit 2 Millimetern Breite, eine relativ kräftige Schraubenfeder 17 eingelegt, bei einer breiteren Schneide, beispielsweise bei einer Schneide mit einer Breite von 5 Millimetern, eine wesentlich schwächere Schraubenfeder 17, so dass die effektiv an der Schneide zur Verfügung stehende Vorschubkraft mit der Breite der Schneide zunimmt.

Im Extremfall kann auf das Einlegen einer Schraubenfeder 17 ganz verzichtet werden, beispielsweise bei sehr breiten Schneiden, etwa bei einer Breite mit 6 Millimetern.

Auf diese Weise kontrolliert jede Einheit aus stationärem Schaft und Schiebeschaft die effektiv an der Schneide auftretenden Vorschubkräfte selbst, obwohl die von dem Handgriff und dem motorischen Antrieb gelieferte Vorschubkraft immer gleich ist, der Operateur ist entlastet von der Aufgabe einer Justierung der Vorschubkraft und kann außerdem keinen Fehler begehen.

Einem Handgriff 2 können mehrere Einheiten aus stationärem Schaft und Schiebeschaft zugeordnet sein, die unterschiedliche Dimensionierungen und damit auch unterschiedlich kräftige Schraubenfedern 17 aufweisen, diese Einheiten sind dank der lösbaren Festlegung der Einheiten am Handgriff 2 leicht auswechselbar und wählen den jeweils für diese Einheit benötigten Verlauf der Vorschubkraft durch die jeweils eingelegte Schraubenfeder 17 selbst aus.

## Patentansprüche

1. Chirurgische Knochenstanze mit einem mit einem Handstück verbundenen stationären Schaft (5), einem an diesem stationären Schaft (5) längsverschieblich gelagerten Schiebeschaft (6), einem elastischen Federelement (17) und mit einem motorischen Antrieb im Handgriff zum Verschieben des Schiebeschaftes (6) aus einer proximalen Ruhestellung in eine distale Arbeitsstellung mit einer vorgegebenen Vorschubkraft, bei welcher der Schiebeschaft (6) einen Anschlag (18) trägt, der beim Verschieben des Schiebeschaftes (6) in die Arbeitsstellung an dem elastischen Federelement (17) anliegt, das sich am stationären Schaft (5) abstützt und beim Verschieben des Schiebeschaftes (6) der Vorschubkraft des Antriebes entgegenwirkt, **dadurch gekennzeichnet, dass** der Anschlag (18) des Schiebeschaftes (6), das elastische Federelement (17) und die Abstützung (16) des elastischen Federelements (17) am stationären Schaft (5) so angeordnet und dimensioniert sind, dass das elastische Federelement (17) bei der Verschiebung des Schiebeschaftes (6) aus der Ruhestellung in die Arbeitsstellung erst nach einem Teil des Verschiebeweges wirksam wird und der Vorschubkraft entgegenwirkt.

2. Knochenstanze nach Anspruch 1, **dadurch gekennzeichnet, dass** der stationäre Schaft (5) eine Aufnahmekammer (16) für ein als Druckfeder ausgebildetes elastisches Federelement (17) aufweist, in die ein am Schiebeschaft (6) angeordneter Anschlag (18) eintaucht, der beim Vorschieben des Schiebeschaftes (6) das elastische Federelement (17) komprimiert.

3. Knochenstanze nach einem der voranstehenden Ansprüche, **dadurch gekennzeichnet, dass** der stationäre Schaft (5) mit dem Schiebeschaft (6) lösbar mit dem Handgriff (2) verbunden und von diesem abnehmbar ist.

4. Knochenstanze nach Anspruch 3, **dadurch gekennzeichnet, dass** ihr ein Satz von mehreren auswechselbaren stationären Schäften (5) mit Schiebeschäften (6) zugeordnet ist, von denen mindestens ein Teil verschieden dimensionierte und mit unterschiedlichen Vorschubkräften einsetzbare Schneiden (12) trägt, und dass jede Einheit aus stationärem Schaft (5) und Schiebeschaft (6) entweder kein elastisches, der Vorschubkraft entgegen wirkendes elastisches Federelement oder ein elastisches Federelement (17) mit unterschiedlicher Federcharakteristik aufweist, so dass die vom Antrieb erzeugte Vorschubkraft vollständig bzw. entsprechend der jeweiligen Federcharakteristik unterschiedlich reduziert auf die Schneide (12) oder die Schneiden einwirkt.

5. Knochenstanze nach Anspruch 4, **dadurch gekennzeichnet, dass** die Schneide (12) oder die Schneiden der verschiedenen Einheiten unterschiedlich breit sind.

## Claims

1. Surgical bone punch having a stationary shaft (5) joined to a handgrip, a slide shaft (6) mounted for longitudinal displacement on this stationary shaft (5), an elastic spring element (17) and a motorized drive in the handgrip for displacement of the slide shaft (6) from a proximal inoperative position to a distal operative position with a preset forward displacement force, wherein the slide shaft (6) carries a stop (18), which, during displacement of the slide shaft (6) to the operative position, strikes the elastic spring element (17), which is supported on the stationary shaft (5), and, during displacement of the slide shaft (6), counteracts the forward displacement force of the drive, **characterized in that** the stop (18) of the slide shaft (6), the elastic spring element (17) and the support (16) of the elastic spring element (17) on the stationary shaft (5) are so arranged and dimensioned that the elastic spring element (17), during the displacement of the slide shaft (6) from the inoperative position to the operative position, becomes operative and counteracts the forward displacement force only after a portion of the path of displacement.

2. Bone punch in accordance with claim 1, **characterized in that** the stationary shaft (5) has a receiving chamber (16) for an elastic spring element (17) configured as a compression spring, into which receiving chamber a stop (18) arranged on the slide shaft (6) extends and, during forward displacement of the slide shaft (6), compresses the elastic spring element (17).

3. Bone punch in accordance with any one of the preceding claims, **characterized in that** the stationary shaft (5) with the slide shaft (6) is releasably joined to and detachable from the handgrip (2).

4. Bone punch in accordance with claim 3, **characterized in that** a set of several exchangeable stationary shafts (5) with slide shafts (6) is allocated to the bone punch, at least some of these carrying differently dimensioned cutting edges (12) employable with different forward displacement forces, and **in that** each unit consisting of stationary shaft (5) and slide shaft (6) either does not have an elastic spring element counteracting the forward displacement force or has an elastic spring element (17) with a different spring characteristic, so that the forward displacement force generated by the drive acts on the cutting edge (12) or on the cutting edges to the full extent or to a reduced extent which differs in accordance with the respective spring characteristic.

5. Bone punch in accordance with claim 4, **characterized in that** the cutting edge (12) or the cutting edges of the different units are of different width.

## Revendications

1. Poinçonneuse chirurgicale pour os, comprenant un corps allongé stationnaire (5) relié à une pièce de poignée, un corps allongé coulissant (6) monté en coulissement longitudinal sur ce corps allongé stationnaire (5), un élément de ressort élastique (17), et un moyen d'entraînement motorisé dans la poignée pour faire coulisser le corps allongé coulissant (6) d'une position de repos proximale à une position de travail distale avec une force d'avance prédéterminée, poinçonneuse dans laquelle le corps allongé coulissant (6) porte une butée (18) qui, lors du coulissement du corps allongé coulissant (6) dans la position de travail, s'applique contre l'élément de ressort élastique (17) qui pour sa part prend appui sur le corps allongé stationnaire (5) et, lors du coulissement du corps allongé coulissant (6), agit à l'encontre de la force d'avance du moyen d'entraînement,
**caractérisée en ce que** la butée (18) du corps allongé coulissant (6), l'élément de ressort élastique (17) et l'appui (16) de l'élément de ressort élastique (17) sur le corps allongé stationnaire (5) sont agencés et dimensionnés de manière telle, que l'élément de ressort élastique (17), lors du coulissement du corps allongé coulissant (6) de la position de repos à la position de travail, ne devienne actif et n'agisse à l'encontre de la force d'avance qu'après une partie de la course de coulissement.

2. Poinçonneuse pour os selon la revendication 1, **caractérisée en ce que** le corps allongé stationnaire (5) présente une chambre de réception (16) pour un élément de ressort élastique (17) réalisé sous la forme d'un ressort de compression, chambre dans laquelle vient plonger une butée (18) qui est agencée sur le corps allongé coulissant (6) et comprime l'élément de ressort élastique (17) lors de l'avance coulissante du corps allongé coulissant (6).

3. Poinçonneuse pour os selon l'une des revendications précédentes, **caractérisée en ce que** le corps allongé stationnaire (5) avec le corps allongé coulissant (6), est relié de manière amovible à la poignée (2) et peut être retiré de celle-ci.

4. Poinçonneuse pour os selon la revendication 3, **caractérisée en ce qu'**il lui est associé un jeu de plusieurs corps allongés stationnaires (5) interchangeables avec des corps allongés coulissants (6) dont au moins une partie porte des tranchants de coupe (12) de dimensions distinctes et pouvant être utilisés avec des forces d'avance différentes, et **en ce que** chaque unité constituée du corps allongé stationnaire (5) et du corps allongé coulissant (6), soit ne présente pas d'élément de ressort élastique agissant à l'encontre de la force d'avance, soit présente un élément de ressort élastique (17) à caractéristique de ressort différente, de sorte que la force d'avance produite par le moyen d'entraînement agit pleinement ou respectivement de manière différemment réduite conformément à la caractéristique de ressort considérée, sur le tranchant de coupe (12) ou les tranchants de coupe.

5. Poinçonneuse pour os selon la revendication 4, **caractérisée en ce que** le tranchant de coupe (12) ou les tranchants de coupe des différentes unités sont de largeur différente.
